# EUROPEAN PATENT APPLICATION

(11) **EP 4 821 394 A1**
(43) Date of publication of application: **14.10.2026**
(21) Application number: 26382391.6
(22) Date of filing: 23.03.2026
(51) Int. Cl.: A45D 26/00, B65D 81/34

(54) **WAXING KIT WITH SELF-HEATING ELEMENT**

(30) Priority: 07.04.2025 ES 202530634 U
(71) Applicant: GRUPO DRV PHYTOLAB, S.L., 28770 Colmenar Viejo (Madrid) (ES)
(72) Inventor: VIDAL ESMORIS, Raúl, 28770 Colmenar Viejo (Madrid) (ES)
(74) Representative: De la Fuente Fernandez, Dionisio

(57) **Abstract**

The present invention discloses a wax hair removal kit that comprises a container (10) for the exothermic reaction with water; at least one envelope of material (20) with a sealing film that incorporates the compounds that react with water or dissolve in it and that is disposed inside the container (10) for the exothermic reaction; wax beads (40); a plate (30) for containing the wax beads (40); a lid (50) for closing the plate (30) that allows the heat to be maintained in said plate (30), wherein said plate (30) is disposed over the opening of the container (10) for the exothermic reaction; and at least one wooden spatula for spreading the wax on the user's dermis. Wax hair removal kit maintains the fluidity of the wax to be spread on the area to be depilated and at the same time the heat of this does not damage the user's dermis.

## Description

### SUMMARY

The present invention discloses a wax hair removal kit that includes a self-heating element through an exothermic reaction, which provides the necessary energy to heat the wax uniformly with water vapor at an ideal temperature, that is, one that maintains the fluidity of the wax to be spread on the area to be depilated and at the same time the heat of this does not damage the user's dermis.

### TECHNICAL FIELD

Hair removal. Hair removal tools. Hair removal kit. Hair removal wax.

### BACKGROUND OF THE INVENTION

Among the techniques for maintaining smooth, soft, and supple skin, waxing stands out. While it can be somewhat painful, it remains one of the most popular methods for skincare. Waxing removes body hair from the root. It is perhaps the oldest hair removal method. It leaves a feeling of freshness and smoothness for approximately a month, in addition to promoting better skin health. Waxing offers numerous benefits: it can be performed on any part of the body, including intimate areas, and is suitable for both men and women. Greater smoothness: Waxing provides this characteristic to the skin. Longer lasting results: Waxing lasts longer than other hair removal methods. The hair weakens over time; therefore, it grows back less frequently, less abundantly, and less strongly. Natural exfoliation: Waxing the legs removes dead skin cells from the top layer of skin. Commercially available hair removal kits consist of wax, a heater, a spatula, and a stick, where the heater is required to melt the wax by heating or by using a water bath.

The melted wax is then allowed to cool slightly to a fluid temperature, but not too hot to avoid burns. It is then spread with a spatula over the area to be waxed, always in the direction of hair growth. It is left to dry on the skin, and then the end of the wax is grasped with a stick and pulled off in the opposite direction of hair growth. Finally, it is recommended to moisturize the skin with essential oils or hydrating creams after waxing.

The problem with these hair removal kits is that they require a plug-in wax melter or a bowl in which the wax can be heated in a double boiler or microwave. It's essential to constantly monitor the temperature to maintain the wax's fluidity and ensure it's hot enough to avoid burns. Some wax melters, like the one described in utility model ES1014340, include a wax reservoir with the applicator mounted on a single-piece head. This reservoir is housed within a sleeve that is heated externally by an electric element to melt the wax, allowing it to be applied in a fluid or liquid state. The sleeve is complemented by an external casing that may or may not extend into a handle.

However, if you don't have a microwave oven and choose to melt the wax using a double boiler, once the water boils, place the other container with the wax inside and reduce the heat to medium. Stir the mixture with a wooden spatula until it has completely melted, then turn off the heat. Finally, if using a microwave, heat in 10-second intervals, checking and stirring constantly until a smooth, homogeneous mixture is formed.

To avoid this problem with the technique, the present invention discloses a hair removal kit that includes a self-heating element, powered by an exothermic reaction, which provides the necessary energy to heat the wax uniformly to an ideal temperature. This temperature maintains the wax's fluidity for application to the area to be depilated, while ensuring that the heat does not damage the user's skin. This eliminates the need for electrical elements to melt the wax and the constant stirring and turning off of the heat to achieve the ideal temperature. The self-heating element of the invention keeps the wax fluid and uniform at a temperature that does not cause skin damage.

The invention kit includes wax beads, as these are less messy than traditional wax. They are gentler on the skin, as they leave no sticky residue. Furthermore, they don't require high temperatures for application; the wax beads melt at low temperatures, thus preventing the risk of burns.

Once the wax is ready, apply it to the desired area with a wooden spatula and wait for it to dry before removing it. It's important to leave the edges thicker to make it easier to grip and pull off. Once finished, it's advisable to refresh and soothe the area with cool or lukewarm water, which will also help close the pores. As a final step, apply a post-depilatory gel with rosehip and argan oil, which will help hydrate and regenerate the skin after hair removal.

### DESCRIPTION OF THE DRAWINGS

To complement the description being made and to help a better understanding of the characteristics of the invention, according to a preferred example of its practical embodiment, a set of drawings is included as an integral part of said description, in which, for illustrative and non-limiting purposes, the following has been represented:

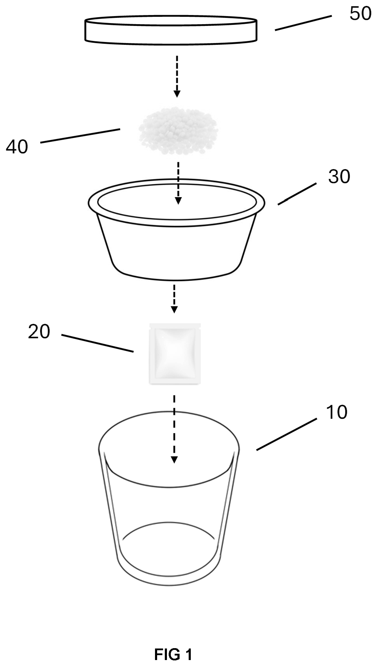

Figure 1 shows an exploded view of the fundamental elements of the hair removal kit of the present invention, showing how the elements are arranged for application: the container (10) for the exothermic reaction with water; the envelope of material (20) that incorporates the compounds that react with water or dissolve in it and that is placed inside the container (10) for the exothermic reaction; the wax beads (40); the plate (30) to hold the wax beads (40); the lid (50) to close the plate (30) that allows the heat to be maintained in said plate (30), where said plate (30) is placed over the opening of the container (10) for the exothermic reaction.

### PREFERRED EMBODIMENT OF THE INVENTION

The present invention shows a wax hair removal kit that allows hair removal at an ideal temperature, where the heating of the wax is carried out by means of a self-heating element based on an exothermic reaction that provides the necessary energy to melt the wax at a temperature that keeps it fluid and at the same time does not cause damage to the user's dermis.

The kit of the invention comprises a container (10) for the exothermic reaction with water, which allows wax beads (40) to be heated by the heat released by the reaction and emitted in the form of water vapor. At least one envelope of material (20) with a sealing film incorporates the compounds that react with or dissolve in water, preferably aluminium powder, sodium bicarbonate, or calcium oxide. Alternatively, magnesium oxide or copper and zinc sulphate powder can also be used. In some embodiments, anhydrous calcium chloride is also used. In this last alternative, no chemical reaction occurs; instead, the heat of dissolution of the product in water is generated.

The envelope of material (20) is made of non-woven fabric or can also be made of paper, cardboard, or similar material and is placed at the bottom of the container (10) for the exothermic reaction with 15 ml of water, which initially dissolves the sealing film. The reaction principle is that the heating ingredients react with water at room temperature through an exothermic reaction to release heat. The heated water produces enough steam to heat to 60-98 °C; the heating time will usually last about 10-15 minutes.

The exothermic reaction is CaO(s) + H₂O(I) → Ca(OH)₂ (s). The heat released by this reaction is 62.3 kJ/mol. It generates heat in 3 minutes and remains hot for 20 minutes.

Or, if copper(II) sulphate is used: CuSO₄ (s) + Zn(s) → ZnSO₄ (s) + Cu(s)

The kit also includes a plate (30) to hold the wax beads (40) to be melted and a lid (50) to retain the heat generated by the steam from the reaction vessel in the plate (30). The plate (30) is placed over the opening of the exothermic container (10) and sealed with the lid (50), so that the heat generated in the container (10) is released and heats the plate (30) containing the wax beads (40). This allows the wax beads (40) to be melted in a controlled manner. The kit also includes at least one wooden spatula for spreading the wax on the user's skin.

The container (10), the plate (30), and the lid (50) are all made of disposable or biodegradable plastic. They can be made from disposable plastic bags or paper bags, cardboard, Kraft paperboard, or corrugated cardboard, and will be customized according to the product design.

The kit of the invention allows the user to melt enough wax beads (40) by placing the envelope of material (20) containing the reaction components into the container (10) and adding 15 ml to 20 ml of water to the container (10). The generated heat evaporates some of the water, heating the plate (30) containing the wax beads (40) with its lid (50). This melts the wax beads (40) to an ideal temperature after 10 to 15 minutes; that is, the wax is fluid but not too hot to apply to a specific part of the body without damaging the skin. Therefore, there is no need to use an electrical energy source (wax warmer, microwave). The product is thus in the "ideal" state for application and hair removal, whether by an expert or a beginner.

Once the wax is ready, apply it to the desired area with a wooden spatula and wait for it to dry before removing it. Remember to leave the edges thicker for easier gripping and pulling.

Once the hair removal process is complete, refresh and soothe the area with cool or lukewarm water, which will also help close the pores. As a final step, apply a post-hair removal gel with rosehip and argan oil, which will help hydrate and regenerate the skin after hair removal

## Claims

1. Waxing kit with self-heating elements **characterized in that** it comprises a container (10) for the exothermic reaction with water; at least one envelope of material (20) with a sealing film that incorporates the compounds that react with water or dissolve in it and that is disposed inside the container (10) for the exothermic reaction; wax beads (40); a plate (30) for containing the wax beads (40); a lid (50) for closing the plate (30) that allows the heat to be maintained in said plate (30), wherein said plate (30) is disposed over the opening of the container (10) for the exothermic reaction; and at least one wooden spatula for spreading the wax on the user's dermis.

2. Waxing kit with self-heating elements according to claim 1 **characterized in that** the envelope of material (20) is made of a non-woven fabric material.

3. Waxing kit with self-heating elements according to claim 1 **characterized in that** the envelope of material (20) is made of paper.

4. Waxing kit with self-heating elements according to claim 1 **characterized in that** the envelope of material (20) is made of cardboard.

5. Waxing kit with self-heating element according to claim 1 **characterized in that** the compounds that react with water are aluminium powder, sodium bicarbonate and calcium oxide.

6. Waxing kit with self-heating element according to claim 1 **characterized in that** the compound that reacts with water is magnesium oxide.

7. Waxing kit with self-heating element according to claim 1 **characterized in that** the compounds that react with water are copper and zinc sulphate powder.

8. Waxing kit with self-heating element according to claim 1 **characterized in that** the compound that dissolves in water is anhydrous calcium chloride.

9. Waxing kit with self-heating element according to claim 1 **characterized in that** the reaction container (10) as well as the plate (30) and the lid (50) are made of disposable plastic material or biodegradable plastic material.

10. Waxing kit with self-heating element according to claim 1 **characterized in that** the reaction container (10) as well as the plate (30) and the lid (50) are made of paper, cardboard, Kraft cardboard or corrugated cardboard.
